(19) Europäisches Patentamt / European Patent Office / Office européen des brevets

(11) **EP 2 620 445 B1**

(12) **EUROPEAN PATENT SPECIFICATION**

(45) Date of publication and mention
of the grant of the patent:
**12.04.2017 Bulletin 2017/15**

(21) Application number: **11826305.2**

(22) Date of filing: **09.09.2011**

(51) Int Cl.:
*C07K 7/08* *(2006.01)*  *C12N 15/11* *(2006.01)*
*C12N 15/63* *(2006.01)*  *A61K 38/10* *(2006.01)*
*A61P 1/10* *(2006.01)*

(86) International application number:
**PCT/CN2011/001541**

(87) International publication number:
**WO 2012/037778 (29.03.2012 Gazette 2012/13)**

(54) **POLYPEPTIDE TF1 FOR INHIBITING TYPE 2 SHIGA TOXIN ACTIVITY, ENCODING GENE FOR SAME AND USE THEREOF**

POLYPEPTID TF1 ZUM HEMMEN VON TYP 2 SHIGATOXIN-AKTIVITÄT, KODIERUNGSGEN DAFÜR UND VERWENDUNG DAVON

POLYPEPTIDE TF1 INHIBANT L'ACTIVITÉ DE LA TOXINE SHIGA DE TYPE 2, SON GÈNE CODEUR ET SON UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.09.2010 CN 201010290461**

(43) Date of publication of application:
**31.07.2013 Bulletin 2013/31**

(73) Proprietor: **Institute Of Microbiology And Epidemiology,
Academy Of Military Medical Sciences, Chinese Pla
Fengtai District
Beijing 100071 (CN)**

(72) Inventors:
• **WANG, Hui**
**Beijing 100071 (CN)**
• **LI, Tao**
**Beijing 100071 (CN)**
• **WANG, Qin**
**Beijing 100071 (CN)**
• **TU, Wei**
**Beijing 100071 (CN)**
• **HOU, Xiaojun**
**Beijing 100071 (CN)**

(74) Representative: **Hoyng Rokh Monegier LLP
Rembrandt Tower, 31st Floor
Amstelplein 1
1096 HA Amsterdam (NL)**

(56) References cited:
**EP-A1- 1 782 820      CN-A- 101 016 332
CN-A- 101 016 332      CN-A- 102 040 654**

• **MIURA Y ET AL: "A globotriaosylceramide (Gb3Cer) mimic peptide isolated from phage display library expressed strong neutralization to Shiga toxins", BIOCHIMICA ET BIOPHYSICA ACTA (BBA) - GENERAL SUBJECTS, ELSEVIER, AMSTERDAM, NL, vol. 1760, no. 6, 1 June 2006 (2006-06-01) , pages 883-889, XP025015008, ISSN: 0304-4165, DOI: 10.1016/J.BBAGEN.2006.03.018 [retrieved on 2006-06-01]**
• **NISHIKAWA KIYOTAKA ET AL: "A multivalent peptide library approach identifies a novel Shiga toxin inhibitor that induces aberrant cellular transport of the toxin", FASEB JOURNAL, FED. OF AMERICAN SOC. FOR EXPERIMENTAL BIOLOGY, US, vol. 20, no. 14, 1 December 2006 (2006-12-01), pages 2597-2599, XP002536895, ISSN: 0892-6638**

**(Cont. next page)**

- **BAO SHI-ZHONG ET AL: "Screening of short peptides binding to StxB by phage-display library", WEISHENGWU XUEBAO, vol. 46, no. 5, October 2006 (2006-10), pages 749-752, XP009175625, ISSN: 0001-6209**
- **HAN ZHAOZHONG ET AL: "Screening and identification of receptor antagonist for shiga toxin from random peptides displayed on filamentous bacteriophages", SCIENCE IN CHINA SERIES C LIFE SCIENCES, vol. 42, no. 1, February 1999 (1999-02), pages 43-49, XP9175626, ISSN: 1006-9305**
- **YAMADA Y ET AL: "Design of multifunctional peptides expressing both antimicrobial activity and shiga toxin neutralization activity", BIOORGANIC & MEDICINAL CHEMISTRY, PERGAMON, GB, vol. 14, no. 1, 1 January 2006 (2006-01-01), pages 77-82, XP027992254, ISSN: 0968-0896 [retrieved on 2006-01-01]**

- **HAN ZHAOZHONG: 'Screening inhibitor of Shiga toxin-receptor from phagedisplay peptide library.' SCIENCE IN CHINA (SERIES C) vol. 29, no. 2, 15 April 1999, pages 151 - 156**
- **BAO, SHIZHONG ET AL.: 'Screening of short peptides binding to StxB by phage-display library.' ACTA MICROBIOLOGICA SINICA vol. 46, no. 5, 04 October 2006, pages 749 - 752**

Sequence list

<110> Institute of Microbiology and Epidemiology, Academy of Military
Medical Sciences, PR China
<120> Polypeptide TF1 for Inhibiting Type 2 Shiga Toxin Activity, Encoding
Gene for Same and Use Thereof
<160> 2
<210> 1
<211> 12
<212> PRT
<213> Artificial Sequence
<220>
<223>
<400> 1

                Thr Phe Asn Met Trp Leu Pro Thr Phe Asn Gln Trp
                1               5                   10

<210> 2
<211> 12
<212> PRT
<213> Artificial Sequence
<220>
<223>
<400> 2

                Trp Ala Pro Trp Tyr Ser Phe Thr Ser Tyr His Leu
                1               5                   10

**Description**

**Technical Field**

The present invention involves a polypeptide TF1 inhibiting the activity of type-2 Shiga toxin (Stx2), the gene encoding the same and the use thereof.

**Background**

Shiga toxin (Stx), also known as Shiga-like toxin (Slt), is a class of bacterial exotoxins having enterotoxicity, cytotoxicity and neurotoxicity. It is produced by enteric pathogens and divided into type-1 and type-2 (Stx1 and Stx2). Stx, as the key virulence factor of enteric pathogens such as *Shigella dysenteriae, Vibrio cholera* and *Escherichia coli,* may lead to several serious complications such as hemorrhagic colitis (HC), thrombotic thrombocytopenic purpura (TTP) and hemolytic uremic syndrome (HUS) which has a high fatality rate. Both of epidemiological and clinical data have demonstrated that Stx2 is more correlated with infectious complications especially HUS than Stx1 and thus regarded as the target for drugs.

At present, as for the infection caused by Stx-producing pathogens, there is not any specific and effective prophylactic or therapeutic drug for the first-aid treatment. Antibiotics commonly used in clinical treatment have given rise to the emergence of multiple drug-resistant bacterial strains, making such therapies noneffective. More seriously, the use of antibiotics may trigger the excessive release of Stx due to the rupture of bacteria, further increasing the risk of developing complications. Therefore, antibiotics should be used with cautions in the treatment of Stx-associated diseases and a specific drug should be developed as a novel effective method.

Stx2 (also known as Slt2) is composed of a single A subunit (Stx2A) and five B subunits (Stx2B). The Stx2B binds to globotriaosylceramide (Gb3) receptor on the surface of eukaryotic cells, and then Stx2A binds to 28S rRNA to cause cytopathy. Therefore, the binding of Stx2B to receptor Gb3 is the initial key step for toxicity. If such binding is blocked, the toxicity of Stx2 can be substantially inhibited. Studies have been mainly focused on the toxin receptor analogues and antibody therapy. Nishikawa reported that polysaccharide compounds which could bind to Stx had a potential therapeutical value (Nishikawa K, Matsuoka K, Watanabe M, et al., Identification of the optimal structure required for a Shiga toxin neutralizer with oriented carbohydrates to function in the circulation. J Infect Dis, 2005, 191: 2097-2105). Natori Y. also reported a treatment case associated with receptor Gb3 in 2002 (Natori Y. New drugs that prevent cytotoxicity of Shiga toxins. Nippon Rinsho. 2002, 60(6): 1131-1137) involving a new preparation that prevents the spread of toxin in gastrointestinal tract and a water-soluble preparation which suppresses Stx cytotoxicity in the circulation. However, the complex preparation process and side-effects of receptor-based polysaccharide compounds have restricted their development. Recently, some researchers reported that probiotics expressing a mimic of the receptor could be used in the treatment of Stx-associated diseases due to its significant neutralizing effect on the toxin (Paton JC, Rogers TJ, Morona R, Paton AW. Oral administration of formaldehyde-killed recombinant bacteria expressing a mimic of the Shiga toxin receptor protects mice from fatal challenge with Shiga-toxigenic Escherichia coli. Infect Immun 2001, 69: 1389-1393). However, whether this approach is safe or not needs to be further tested. As for the antibody therapy, the monoclonal antibody reported by Sheoran et al. can block the binding between Stx2 and cell receptors and further neutralize the toxicity of Stx2 (Sheoran AS, Chapman-Bonofiglio S, Harvey BR, et al. Human antibody against shiga toxin 2 administered to piglets after the onset of diarrhea due to Escherichia coli 0157: H7 prevents fatal systemic complications. Infect Immun 2005; 73:4607-4613). However, it can be clinically used only if preparation technology of antibodies is improved.

As a drug, a peptide compound is gaining more and more attentions due to its technical advantages such as the small molecular weight, relatively simple structure, mass production with low costs by chemical synthesis or genetic engineering methods, identified functions, low immunogenicity, slight side-effects, high security, diversity of administration routes and easy absorption. Thus, small-molecule peptides have become one of the most important sources for drug screening (Yuexi Li, Peitang Huang. Application of polypeptides in biological drugs and diagnosis reagents. Chinese Journal of Biochemical Pharmaceutics, 2001, 22 (4): 208-210). It is feasible to use a phage peptide library which contains a large quantity of peptides to screen for polypeptides having inhibitory effects on a toxin. However, peptide-based inhibitor against Stx has been rarely reported recently. The related literatures are mainly studies of Nishikawa K in 2006 (Nishikawa K, Watanabe M, Kita E, et al. A multivalent peptide library approach identifies a novel Shiga toxin inhibitor that induces aberrant cellular transport of the toxin. Faseb J 2006; 20:2597-2599) and studies of Miura Y (Miura Y, Sakaki A, Kamihira M, Iijima S, Kobayashi K. A globotriaosylceramide (Gb3Cer) mimic peptide isolated from phage display library expressed strong neutralization to Shiga toxins. Biochem Biophys Acta 2006; 1760:883-889).

**Summary of Invention**

The aim of the present invention is to provide a polypeptide which inhibits the activity of type-2 Shiga toxin.

The polypeptide of the present invention is named TF1 (also called P1) and its amino acid sequence is represented by Sequence 1 in the sequence list.

Another aim of the present invention is to provide a modified polypeptide.

The modified polypeptide provided herein is obtained by chemically or biologically modifying the above-mentioned TF1 at its N terminal and/or C terminal.

Further, the chemical modification can be acetylation or aminoacylation.

The modified peptide can be a linear peptide or a branched peptide.

The gene coding the above polypeptide is also embodied in the protection scope of this invention.

The recombinant vector, recombinant bacteria or transgenic cell line containing the gene above is also embodied in the protection scope of this invention.

The use of the above-mentioned polypeptide or modified peptide in the preparation of drugs for preventing and/or treating diseases caused by Stx2 or Stx2-producing pathogens is also embodied in the protection scope of this invention.

The pathogens mentioned above are Stx2-producing *Escherichia coli, Shigella dysenteriae* or *Vibrio cholerae* strains.

Specifically, the Stx2-producing *Escherichia coli* is Enterohemorrhagic *E. coli* 0157.

**Brief Description of the Drawings**

Figures 1A and 1B show the mass analysis result of synthesized peptide TF1 (also called P1), wherein Figure 1A shows the image of mass spectrometry and Figure 1B shows the image of high performance liquid chromatography (HPLC).

Figures 2A and 2B show the mass analysis (at 220 nm) result of synthesized peptide WA8 (also called P8), wherein Figure 2A shows the image of mass spectrometry and Figure 2B shows the image of high performance liquid chromatography.

Figure 3 shows the binding profile between Stx2B and a gradient concentration of polypeptides (P1, P8). The binding amount gradually increases as the concentration of P1 (or P8) increases.

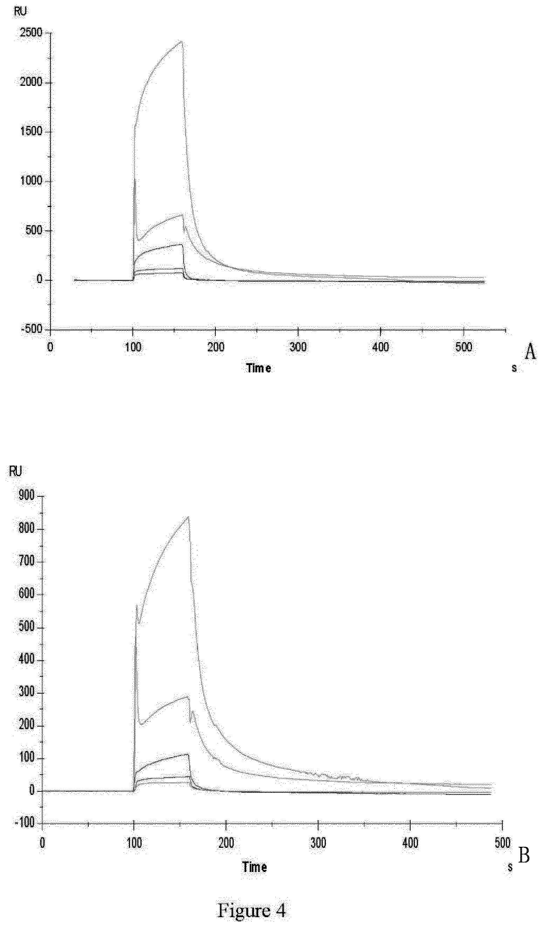

Figures 4A and 4B are graphs showing binding dynamics between the polypeptides and Stx2, wherein the BIAcore system dynamically analyzes the specific binding between Stx2 and TF1 (also called P1) (or WA8 which is also called P8). The lowest curve represents the lowest concentration and the highest curve represents the highest concentration. The concentration of P1 in Figure 4A (KD: 1.25e-6) ranges from 0.96 to 15.38 $\mu$mol/L and the concentration of P8 in Figure 4B (KD: 2.48e-5) ranges from 2.44 to 39.125 $\mu$mol/L, suggesting that polypeptides TF1 and WA8 can specially bind to Stx2 in a dose dependent manner.

Figure 5 shows the inhibitory effect of TF1 (also called P1) and WA8 (also called P8) on cytotoxicity *in vitro* observed by a light microscope (x200). Hela cells are added after incubation. A: Control group (PBS). B: Stx2 group. C: Stx2 + polypeptide (P1 or P8).

Figure 6 shows the protective effect of the polypeptide against 5LD50 in mice. The mice were intraperitoneally administered with polypeptide and then 20 minutes later with Stx2 (5LD50). Figure 6A shows the effect of P1 and Figure 6B shows the effect of P8, both polypeptides being administered in the range of 0.7 mg/kg-11 mg/kg.

**Detailed Description of the Invention**

The present invention will be further described in combination with the following specific examples but the present invention is not limited to these examples.

The methods used in the examples are common ones if not indicated otherwise.

**Example 1. Modification, chemical synthesis and binding activity analysis of the polypeptides TF1 (also called P1) and WA8 (also called P8)**

1. Modification and synthesis of the polypeptides TF1 (also called P1) and WA8 (also called P8)

1) Screening for the sequences of TF1 and WA8

Preparation of Stx2B: The stx2B gene was amplified from *E. coli* EHEC 0157: H7 (purchased from ATCC) using 5'-catatggctaaaggtaaaattgag-3' and 5' gcggccgcgcctcagtcatcagtcatt-3' as primers. The amplified fragment was sequenced in pEASY-T1 and its nucleotide sequence was confirmed to be No.1353327-1353533 of the nucleotide of Genbank NC_002655. Subsequently, the amplified product was inserted into pET22b+ (purchased from Novagen) digested with restriction endonucleases *Nde* I and *Not* I to constract a recombinant plasmid pET22b-*stx2B*. The plasmid pET22b-*stx2B* was transformed into *E. coli* BL21 (DE3) strain (purchased from Transgen) to obtain a recombinant strain pET22b-*stx2B*/BL21 (DE3). Clones of the recombinant strain were propagated in LB medium containing 100 $\mu$g/mL of ampicillin and incubated in water bath at 37°C with shaking overnight. Then, the culture was subcultured into erlenmeyer flasks in a ratio of 1:100 to further grow the strains. When the culture grew to OD600 of 0.6, IPTG was added to a final concentration of 1 mmol/L. The inducible expression continued for 5 hours, leading to the effective expression of Stx2B. Cells were treated by ultrasonication and then centrifuged at 10,000 r/min for 10 min. Inclusion bodies were collected and dissolved in 6 mol/L guanidine hydrochloride in PBS. Thereafter, denatured proteins were transferred into a dialysis bag and dialyzed in a series of Tris buffer (10 mmol/L Tris, 1 mmol/L EDTA, 0.3 mol/L arginine, 1 mmol/L reduced glutathione, 0.25 mmol/L oxidized glutathione, 10% of glycerol, 50 mmol/L NaCl, pH 8.0) with decreasing concentrations of urea (6, 4, 2, 1, 0 mol/L). The last buffer for dislysis was 10 mmol/L Tris, pH 8.0. Denatured proteins were purified via linear elution using a Q Sepharose column with 10 mmol/L Tris - 1 mol/L NaCl as the eluent. The details of this experiment has been published (Tu W, Cai K, Gao X, Xiao L, Chen RC, Shi J, Liu H, Hou XJ, Wang Q, Wang H, Improved production of holotoxin Stx2 with biological activities by using a single-promoter vector and an auto-induction expression system. Protein Expr. Purif. 2009, 67(2):169-174).

Phage display technique was used to screen for polypeptides specifically binding to Stx2B from the random linear twelve-peptided library based on their affinity to the recombinant Stx2B. Two sequences most frequently occurred were picked up from the screened peptides and named TF1 (also P1) and WA8 (also P8). Subsequently, the physicochemical properties of TF1 and WA8 were predicted by the protparam tool (http://www.expasy.org/tools/ protparam.html). The results indicated that TF1 was composed of 12 amino acids and could be represented by the formula of $C_{77}H_{101}N_{17}O_{18}S_1$, wherein the molecular weight was 1584.8 and the theoretic pI was 5.19. In addition, WA8 was found to be composed of 12 amino acids and could be represented by the formula of $C_{79}H_{96}N_{16}O_{18}$, wherein the molecular weight was 1557.7 and the theoretic pI was 6.74.

2) Chemical modification and synthesis *in vitro*

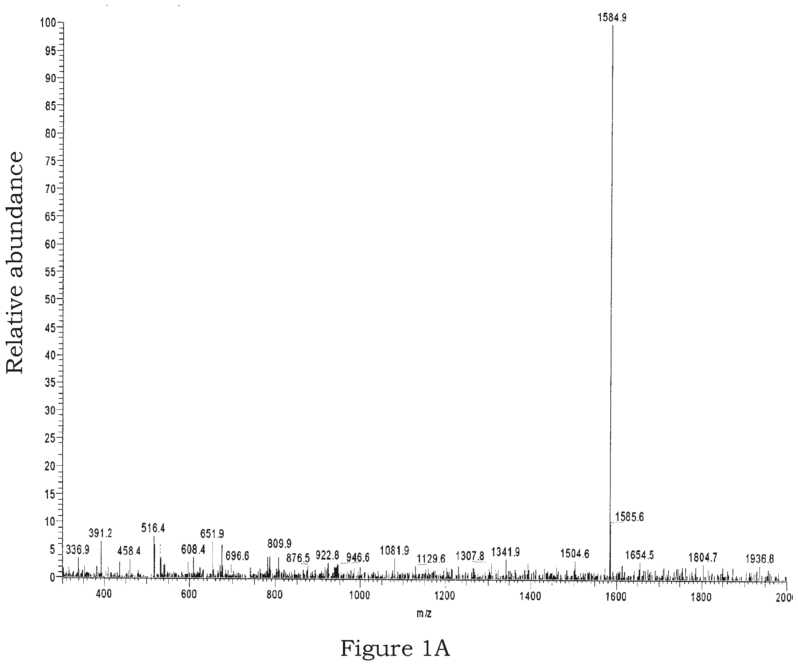

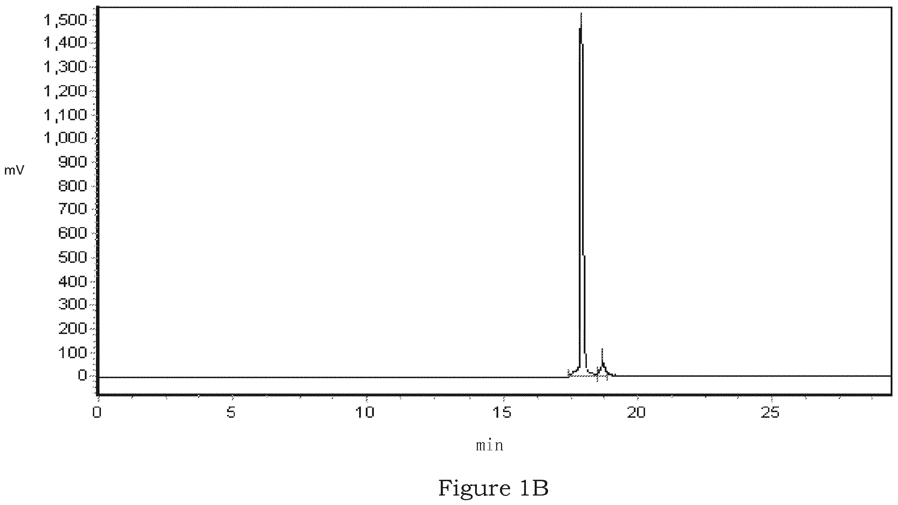

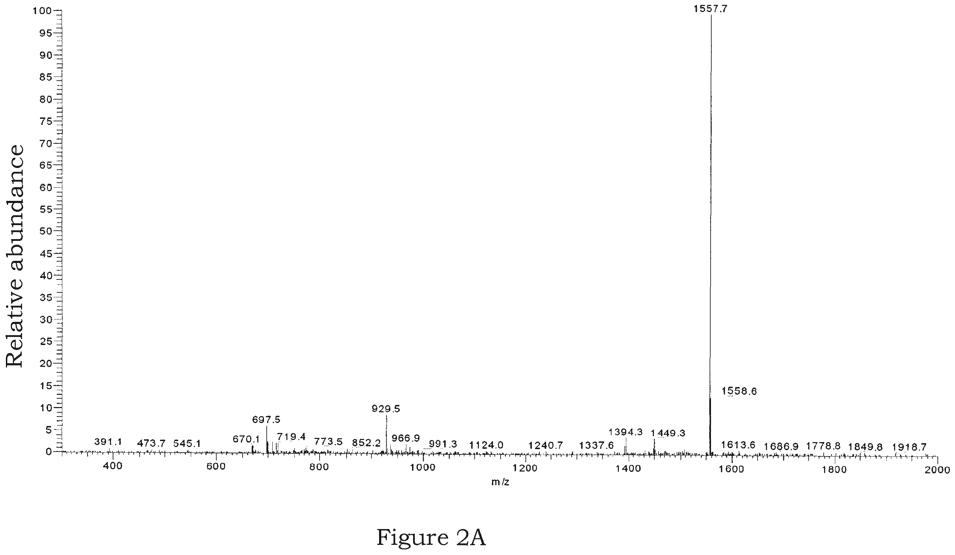

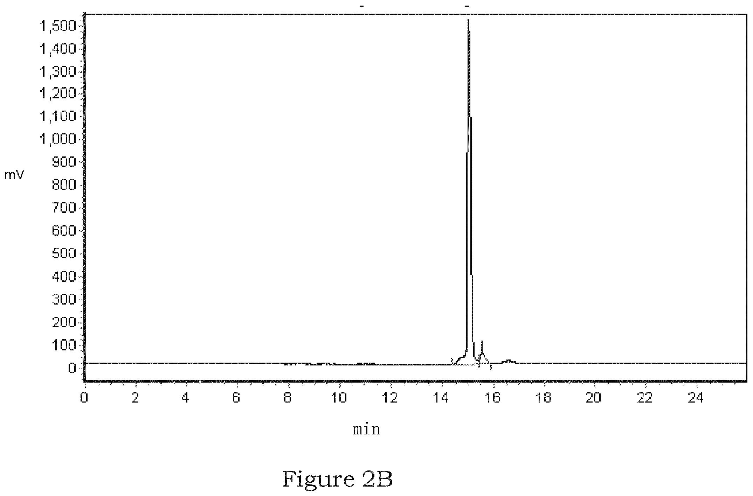

The polypeptides TF1 and WA8 were acetylated at N-terminal without changing their primary structures, which would improve the water-solubility and stability. Such a chemical modification was done by Shanghai Bootech Bioscience&Technology Co., Ltd. The peptides were synthesized by solid phase synthesis and then subjected to perform mass spectra and HPLC. As shown in Fig. 1 and Fig. 2, the molecular weight of TF1 was 1584.9 (Figure 1A, mass spectra) which conformed with the theoretical value and its purity was 95.1% (Figure 1B, HPLC); the molecular weight of WA8 was 1557.7 (Figure 2A, mass spectra) which substantially conformed with the theoretical value and its purity was 95.3% (Figure 2B, HPLC).

The amino acid sequence of TF1 (also called P1) was represented by Sequence 1 in the sequence list and that of WA8 (also called P8) was represented by Sequence 2.

2. Analysis of binding activity of polypeptides TF1 (also called P1) and WA8 (also called P8)

The biosensor system BIAcore 3000, CM5 chip and corresponding reagents were purchased from GE and the HRP-conjugated anti-histidine antibody was purchased from Pierce (ELISA titer: 1:2000). *Escherichia coli* BL21 (DE3) plysS Chemically Competent Cell, *E. coli* DH5$\alpha$ and pEASY-T1 simple cloning vector were purchased form Beijing TransGen Biotech (China). Taq DNA polymerase, T4 DNA ligase and restriction endonucleases were obtained from New England Biolabs (Beijing, China). PCR primers were synthesized by Beijing Sunbio Tech Co. Ltd. Plasmid mini-kit and Gel extraction kit were purchased from Beijing Biomed Co. Ltd. HisTrap FF column (5 ml) were purchased from GE Healthcare (Beijing, China). Weight 8-10 kDa cut-off dialysis membrane tubing was purchased from Beijing Kehaijunzhou Biotechnology Development Center. The expression vector pET32a was purchased from Novagen.

1). Enzyme-linked immunosorbent assay (ELISA) analysis of interaction between the polypeptides and Stx2B.

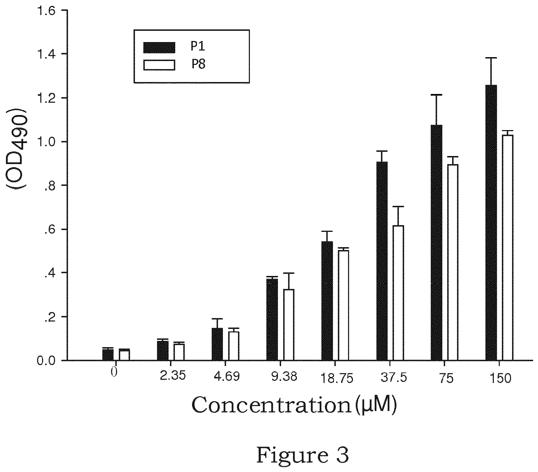

The ELISA microtiter plates were coated overnight with a gradient concentration of polypeptides at 4 °C (BSA as a negative control) and washed with PBST. Then it was blocked with 3% BSA for 1 hour and washed with PBST. Stx2B prepared in step 1 was added and then incubated at 37°C for 1 hour. Following PBST washing, anti-histidine antibody was used to test Stx2B which bound to the polypeptides. After the colorimetric reaction, the absorption at 450 nm was recorded. As shown in Figure 3, as the concentration of polypeptide (P1 or P8) increased, the amount of polypeptides binding to Stx2B increased gradually. More P1 bound to Stx2B than WA8 at the same concentration.

2). Surface Plasmon Resonance technology (SPR)-based kinetics analysis of the specific binding of TF1 and/or WA8 to Stx2

Preparation of Stx2: The *stx2* gene was amplified from *E. coli* EHEC 0157: H7 using 5'-GGGGGGAATTCATGAAGTGTATATTATTTAAATGGG-3' (primer 1) and 5'-TTTTTTGTCGACTTAGTGGTGGTGGTGGTGGTGGTCATTATTAAACTGCACTTCAG-3' (primer 2) as primers. The amplified fragment was subjected to sequencing in pEASY-T1 and its nucleotide sequence was confirmed to be No.1353327-1353533 of the nucleotide of Genbank NC_002655. A recombinant strain pET32a-*stx2*/plysS was constructed, which contained two open reading frames with a single promoter. Specifically, the amplified product was inserted into pET32a digested with restriction endonucleases *EcoR* I and *Sal* I to construct a recombinant plasmid pET32a-*stx2*. Then this plasmid was transformed into *E. coli* BL21 (DE3) plysS strain (purchased from Transgen) to obtain a recombinant strain pET32a-*stx2*/plysS.

The recombinant plasmid pET32a-*stx2* had one T7 promoter and the full-length clone of *stx2* contained two open reading frames (*stx2a* and *stx2b*). IPTG was added to a final concentration of 1 mmol/L. Thereafter, the cells were cultivated at 30°C for 5 hours in water bath while shaking, leading to the highly effective expression of Stx2. Stx2 with 90% purity or more was obtained after treatment in HisTrap Ni column and the Stx2 showed a biological activity. Details of related experiments have been published (Tu W, Cai K, Gao X, Xiao L, Chen RC, Shi J, Liu H, Hou XJ, Wang Q, Wang H, Improved production of holotoxin Stx2 with biological activities by using a single-promoter vector and an auto-induction expression system. Protein Expr. Purif. 2009, 67(2):169-174).

The specific binding of the polypeptides to the prepared Stx2 was tested by using surface plasmon resonance (SPR) technology in BIAcore biosensor system. Specifically, Stx2 (2235.9 RU) was coupled to the Fc4 in CM5 chip, activated by EDC/NHS and blocked by ethanolamine. The binding kinetics between polypeptides of different concentrations (double diluted) and Stx2 was detected and was then shown in curves. Detection was performed at 20 $\mu$L/min and the dynamic binding process of polypeptides (TF1 or WA8) to Stx2 was observed from Sensorgrams. Analysis was performed by BIAevaluation 3.0, Ka and Kd were respectively obtained from the association and dissociation parts of the curve. The data was quite different for polypeptides of different concentrations. Ka, Kd, KA and KD were calculated based on analysis reports according to the following formulae:

$$Rmax = (MW_{analyte}/MW_{ligand}) \times R_{ligand} \times S_{valence} \qquad (1)$$

$$Ka = (dR/dt)/t \qquad (2)$$

$$Kd = (dt/dR)/R \qquad (3)$$

$$KD = Kd/Ka = C \times (Rmax-Req)/ Req \qquad (4)$$

R: SPR signal at a time point (unit: RU). Req: Analyte stable binding level (unit: RU). Rmax: Peptide maximum binding capacity. t: time. dR/dt: SPR signal conversion rate. C: Peptide concentration.

As shown in Figure 4, the concentration of P1 ranged from 0.96 to 15.38 $\mu$M and that of P8 ranged from 2.44 to 39.125 $\mu$M. The affinity constant KD for P1 binding to Stx2 was calculated to be $1.25 \times 10^{-6}$ M and that for P8 binding to Stx2 was $2.48 \times 10^{-5}$ M.

**Example 2. Inhibitory effect of TF1 or WA8 on cytotoxicity *in vitro* and blocking effect of TF1 or WA8 on the binding of toxin to target cells**

Hela cells, pancreatin and antibodies were purchased from Hyclone Laboratories Inc., DMEM medium, fetal bovine serum, MTT and FITC were respectively from GIBCO Inc., Biochorm Ltd., sigma Inc. and Laibao Inc. Cell culture flasks and plates were purchased from CORNING Inc. $CO_2$ incubator, inverted microscope, flow cytometer were respectively the products of Sanyo Inc. (Japan), Olympus Inc. (Japan) and Coulter Inc.

1. Cytotoxicity neutralization assay

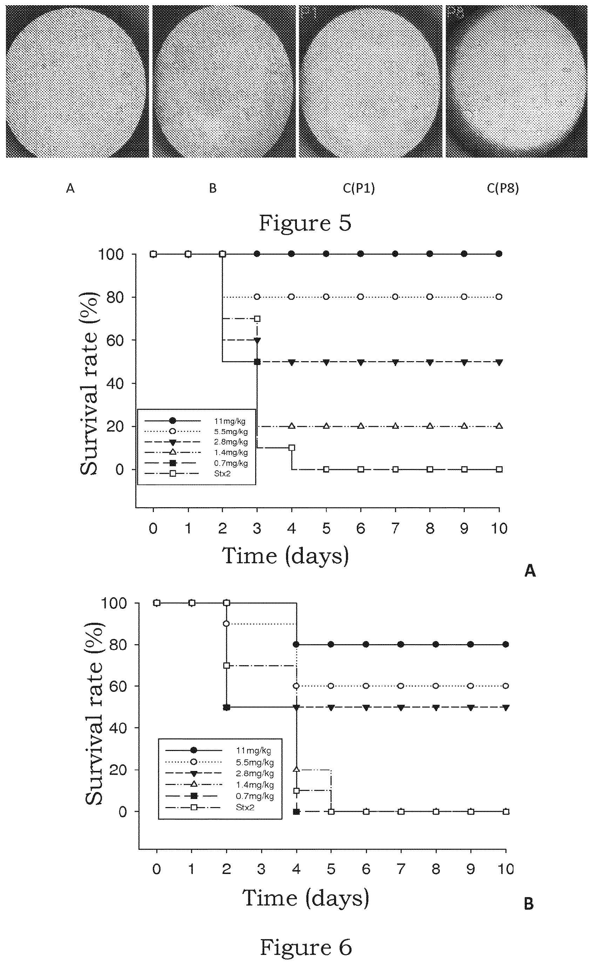

Hela cells at logarithmic phase were collected whose concentration was then adjusted to a certain extent. 100 $\mu$l of cell culture was injected into 96-well microtiter plates and the density of cells in each well was adjusted to $10^4$/well (wells at periphery were filled with sterile PBS). Plates were incubated overnight at 37°C with 5% $CO_2$. Subsequently, 100 $\mu$l of polypeptides (having a final concentration of 150 $\mu$M) were mixed with 2.5 ng of Stx2 (5CD50). Thereafter, the mixture was incubated at 4°C overnight. Meanwhile, two control groups were arranged, one added with only PBS as the normal control and the other added only with Stx2. The mixture was added into cells cultivated in 96-well microplates and incubated in 5% $CO_2$ at 37°C for 72 hours, and then cells were observed using inverted microscope. Culture fluid was discarded and cells were washed carefully with PBS for 2-3 times. 50 $\mu$l of MTT (5 mg/ml) was added into each well and cultivation continued for another 4 hours. Subsequently, culture fluid was carefully discarded and 150 $\mu$l of DMSO was added in each well. Plates were gently shaken in a shaker for 5 min so that crystals were sufficiently dissolved. Absorbance was measured at $OD_{570}$ using a microplate reader. The inhibition rate was calculated according to the following formula: Inhibition rate% = ($OD_{570}$(peptide+Stx2) - $OD_{570}$(Stx2)) / ($OD_{570}$(normal control) - $OD_{570}$(Stx2)). As shown in Figure 5, the inhibition rate of P1 was higher than that of P8 if their concentrations were the same. When Stx2 (5xCD50) was added in an amount exceeding the lethal dose, the IC50 (half maximal inhibitory concentration) of P1 was 180 $\mu$M and that of P8 was 193 $\mu$M. In addition, the inhibition rate reached 82% when the concentration of P1 was 300 $\mu$M and the inhibition rate was 54% when the concentration of P8 was 300 $\mu$M.

2. Analysis on the effect of polypeptide on the binding of Stx2B to cells by Flow cytometry

The HeLa cell selected was Gb3[+] cell and Stx2B was the receptor binding site of Stx2. Stx2B specifically bound to HeLa cell via receptor Gb3. Based on such cell model, the blocking effect of the polypeptide on the binding of Stx2B to HeLa cells can be tested. FITC-Stx2B was prepared by labeling Stx2B with fluorescein FITC. Specifically, the purified Stx2B mentioned above was dialyzed in a carbonate buffer (pH9.2) and FITC in DMSO was added (0.05 mg FITC/ 1 mg protein). The mixture was stirred for 2 hours in dark at RT and dialyzed in a phosphate buffer (pH7.2) during which buffer was renewed until no FITC came out. Thereafter, the prepared FITC-Stx2B (5 $\mu$g) was mixed with polypeptide (100 $\mu$l, final concentration: 300 $\mu$M) and then incubated at 4°C overnight (keep in dark). After about $10^6$ of cells were added, incubation continued at 37°C for 2 hours and the mixture was centrifuged at 1000r/min for 5 min. Subsequently, cells were washed with PBS for two times and then fixed with 40% paraformaldehyde at 4 °C for 10 min. After centrifugation, some cells were suspended in PBS and then detected by flow cytometry. The remaining cells were suspended in PBS and the binding profile of FITC-Stx2B to Hela cells was analyzed by flow cytometry. Three groups were contained in this experiment: negative control group (PBS), FITC-Stx2B group and FITC-Stx2B+polypeptide group. Results (data not shown) demonstrated that 97.22% of HeLa cells treated with FITC-Stx2B showed fluorescence and 52.77% of Hela cells incubated with FITC-Stx2B+polypeptide TF1 (also called P1) showed fluorescence. Thus, TF1 (also called P1) (300 $\mu$mol/L) blocked 45.7% of binding between FITC-Stx2B and HeLa cells. The blocking rate of WA8 (also called P8) (300 $\mu$mol/L) was 24.8 %.

**Example 3. Protective effect of the polypeptide (TF1 or WA8) against Stx2 in mice**

Male Balb/C mice (17-19 g) were provided by the Laboratory Animal Center of the Academy of Military Medical Sciences. TF1 (also called P1, purity>95%, gram) and WA8 (also called P8, purity>95%, gram) were used in this experiment.

I. Protective effect of the polypeptide (TF1 or WA8) against Stx2 lethality in a mice model

1. Preparation of crude Stx2

*E. coli* 0157 (purchased from ATCC) capable of expressing Stx2 was inoculated to LB medium and cultured overnight at 37°C. The culture was diluted in a ratio of 1:100 in 1L of medium the next day and cultured with shaking for another 72 hours. The culture was centrifuged at 5000 rpm for 10 min. Then, the gathered cells were suspended in PBS and ruptured by ultrasonic waves. After being centrifugated at 10,000 rpm for 10 minutes, a solution of saturated ammonium sulfate was added into the supernatant until reaching a concentration of 50%. Subsequently, the precipitate was gathered and dissolved in PBS. After a complete dialysis, a crude product of Stx2 was obtained.

2. Inhibition effect of TF1 and WA8 on Stx2 *in vivo*

The male Balb/C mice (17-19 g) were randomly divided to six groups, each group comprising 20 mice. TF1 (also called P1) or WA8 (also called P8) was intraperitoneally injected into mice. Twenty minutes later, mice were challenged with 50 ng of crude Stx2 (5LD50) by intraperitoneal injection. A placebo control group (see figure 6, STX2) was injected with saline prior to challenging with 50 ng of crude STx2 by intraperitoneal injection. Observation lasted for 10 days and the survival of each group was recorded twice a day. As shown in Figure 6, all the animals in the placebo control group died in Day 4 or Day 5. However, different doses of polypeptide (P1 or P8) provided different extents of protection effect to mice. P1 could totally protect the mice when the dose reached 11 mg/kg (see Fig .6A) and P8 exhibited 80% protection rate at the same concentration (Fig .6B). P1 or P8 exhibited a dose-dependent protection effect. P1 showed a better protection capacity than P8 when Stx2 was added in an amount exceeding the lethal dose.

**Industrial Applications**

The experiments proved that more P1 bound to Stx2 as the concentration of P1 increased. And the affinity constant KD of P1 to Stx2 was $1.25 \times 10^{-6}$ M. It was found in the experiment of cytotoxicity neutralization that the inhibition rate to Stx2 reached 82% when the concentration of P1 was 300 $\mu$M. Further, TF1 (also called P1) (300 $\mu$mol/L) blocked 45.7% of binding of FITC-Stx2B to HeLa cells. In the animal experiment, all the animals in the placebo control group died in Day 4 or Day 5. However, different doses of polypeptides (TF1 or WA8) provided different extents of protection effect to mice. P1 could totally protect mice when the dose reached 11 mg/kg (see Fig .6A) and the polypeptide exhibited a dose-dependent protection effect.

**Claims**

1. A polypeptide, the amino acid sequence of which is represented by Sequence 1 in the sequence list.

2. A modified polypeptide derived from the polypeptide of claim 1, **characterized in that** the modified polypeptide is obtained by performing chemical or biological modification to the polypeptide of claim 1 at its N- and/or C- terminal.

3. The modified polypeptide of claim 2, **characterized in that** the chemical modification is acetylation or aminoacylation.

4. The modified polypeptide of claim 2, **characterized in that** the modified polypeptide is a linear or branched polypeptide.

5. A gene encoding the polypeptide of claim 1.

6. A recombinant vector, a recombinant bacteria or a transgenic cell line carrying the gene of claim 5.

7. The use of the polypeptide of claim 1 or the modified polypeptide of any one of claims 2-4 in the preparation of drugs for preventing and/or treating diseases caused by type-2 Shiga toxin (Stx2) or a Stx2-producing pathogen.

8. The use of claim 7, **characterized in that** the pathogen is a Stx2-producing strain of *Escherichia coli*, *Shigella dysenteriae* or *Vibrio cholerae*.

9. The use of claim 8, **characterized in that** the Stx2-producing *Escherichia coli* is enterohemorrhagic *E. coli* 0157.

10. The polypeptide of claim 1 or the modified polypeptide of any one of claims 2-4 for use for preventing and/or treating diseases caused by type-2 Shiga toxin (Stx2) or a Stx2-producing pathogen.

11. The polypeptide of claim 10, **characterized in that** the pathogen is a Stx2-producing strain of *Escherichia coli*, *Shigella dysenteriae* or *Vibrio cholerae*.

12. The polypeptide of claim 11, **characterized in that** the Stx2-producing *Escherichia coli* is enterohemorrhagic *E. coli* 0157.

**Patentansprüche**

1. Polypeptid dessen Aminosäuresequenz Sequenz 1 in der Sequenzliste entspricht.

2. Modifiziertes Polypeptid, abgeleitet von dem Polypeptid gemäß Anspruch 1, **dadurch gekennzeichnet, dass** das modifizierte Polypeptid durch chemische oder biologische Modifizierung des Polypeptids in Anspruch 1 an dessen N- oder C-terminalem Ende erhalten wird.

3. Modifiziertes Polypeptid gemäß Anspruch 2, **dadurch gekennzeichnet, dass** die chemische Modifizierung ist Acetylierung oder Aminoacetylierung erfolgt.

4. Modifiziertes Polypeptid gemäß Anspruch 2, **dadurch gekennzeichnet, dass** das modifizierte Polypeptid ein lineares oder verzweigtes Polypeptid ist.

5. Gen, welches das Polypeptid gemäß Anspruch 1 kodiert.

6. Rekombinanter Vektor, rekombinantes Bakterium oder eine transgene Zelllinie, die das Gen gemäß Anspruch 5 enthalten.

7. Verwendung des Polypeptids gemäß Anspruch 1 oder des modifizierten Polypeptids gemäß einem der Ansprüche 2-4 zur Herstellung von Medikamenten zur Vorbeugung und/oder Behandlung von Krankheiten, die durch Typ-2 Shiga-Toxin (Stx2) oder einen Stx2-produzierenden Erreger hervorgerufen werden.

8. Verwendung gemäß Anspruch 7, **dadurch gekennzeichnet, dass** der Erreger ein Stx2-produzierender Stamm von *Escherichia coli, Shigella dysenteriae* oder *Vibrio cholerae* ist.

9. Verwendung gemäß Anspruch 8, **dadurch gekennzeichnet, dass** der Stx2-produzierende *Escherichia coli* Stamm der enterohämorrhagische *E. coli* 0157 Stamm ist.

10. Polypeptid gemäß Anspruch 1 oder modifiziertes Polypeptid gemäß einem der Ansprüche 2-4 zur Anwendung bei der Vorbeugung und/oder Behandlung von Krankheiten, die durch Typ-2-Shiga-Toxin (Stx2) oder einen Stx2-produzierenden Erreger hervorgerufen werden.

11. Polypeptid gemäß Anspruch 10, **dadurch gekennzeichnet, dass** der Erreger ein Stx2-produzierender Stamm von *Escherichia coli, Shigella dysenteriae* oder *Vibrio cholerae* ist.

12. Polypeptid gemäß Anspruch 11, **dadurch gekennzeichnet, dass** der Stx2-produzierende *Escherichia coli* Stamm der enterohämorrhagische *E. coli* 0157 Stamm ist.

**Revendications**

1. Polypeptide dont la séquence d'acides aminés est représentée par la séquence 1 dans la liste de séquences.

2. Polypeptide modifié dérivé du polypeptide selon la revendication 1, **caractérisé en ce que** le polypeptide modifié est obtenu par l'accomplissement d'une modification chimique ou biologique sur le polypeptide selon la revendication 1 à son extrémité N- et/ou C-terminale.

3. Polypeptide modifié selon la revendication 2 **caractérisé en ce que** la modification chimique est une acétylation ou une aminoacylation.

4. Polypeptide modifié selon la revendication 2 **caractérisé en ce que** le polypeptide modifié est un polypeptide linéaire ou ramifié.

5. Gène codant le polypeptide selon la revendication 1.

6. Vecteur recombinant, bactérie recombinante ou lignée cellulaire transgénique portant le gène selon la revendication 5.

7. Utilisation du polypeptide selon la revendication 1 ou du polypeptide modifié selon l'une quelconque des revendications 2-4 dans la préparation de médicaments pour prévenir et/ou traiter les maladies causées par la Shiga toxine de type 2 (Stx2) ou un agent pathogène produisant Stx2.

8. Utilisation selon la revendication 7 **caractérisée en ce que** l'agent pathogène est une souche produisant Stx2 de *Escherichia coli, Shigella dysenteriae* ou *Vibrio cholerae.*

9. Utilisation selon la revendication 8 **caractérisée en ce que** *Escherichia coli* produisant Stx2 est *E. coli* entérohémorragique 0157.

10. Polypeptide selon la revendication 1 ou polypeptide modifié selon l'une quelconque des revendications 2-4 destiné à être utilisé pour prévenir et/ou traiter les maladies causées par la Shiga toxine de type 2 (Stx2) ou un agent pathogène produisant Stx2.

11. Polypeptide selon la revendication 10 **caractérisé en ce que** l'agent pathogène est une souche produisant Stx2 de *Escherichia coli, Shigella dysenteriae* ou *Vibrio cholerae.*

12. Polypeptide selon la revendication 11 **caractérisé en ce que** *Escherichia coli* produisant Stx2 est *E. coli* entérohémorragique 0157.

Figure 1A

Figure 1B

Figure 2A

Figure 2B

Figure 3

Figure 4

A               B               C(P1)           C(P8)

Figure 5

A

B

Figure 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **NISHIKAWA K ; MATSUOKA K ; WATANABE M et al.** Identification of the optimal structure required for a Shiga toxin neutralizer with oriented carbohydrates to function in the circulation. *J Infect Dis,* 2005, vol. 191, 2097-2105
- **NATORI Y.** New drugs that prevent cytotoxicity of Shiga toxins. *Nippon Rinsho,* 2002, vol. 60 (6), 1131-1137
- **PATON JC ; ROGERS TJ ; MORONA R ; PATON AW.** Oral administration of formaldehyde-killed recombinant bacteria expressing a mimic of the Shiga toxin receptor protects mice from fatal challenge with Shiga-toxigenic Escherichia coli. *Infect Immun,* 2001, vol. 69, 1389-1393
- **SHEORAN AS ; CHAPMAN-BONOFIGLIO S ; HARVEY BR et al.** Human antibody against shiga toxin 2 administered to piglets after the onset of diarrhea due to Escherichia coli 0157: H7 prevents fatal systemic complications. *Infect Immun,* 2005, vol. 73, 4607-4613
- **YUEXI LI ; PEITANG HUANG.** Application of polypeptides in biological drugs and diagnosis reagents. *Chinese Journal of Biochemical Pharmaceutics,* 2001, vol. 22 (4), 208-210
- **NISHIKAWA K ; WATANABE M ; KITA E et al.** A multivalent peptide library approach identifies a novel Shiga toxin inhibitor that induces aberrant cellular transport of the toxin. *Faseb J,* 2006, vol. 20, 2597-2599
- **MIURA Y ; SAKAKI A ; KAMIHIRA M ; IIJIMA S ; KOBAYASHI K.** A globotriaosylceramide (Gb3Cer) mimic peptide isolated from phage display library expressed strong neutralization to Shiga toxins. *Biochem Biophys Acta,* 2006, vol. 1760, 883-889
- **TU W ; CAI K ; GAO X ; XIAO L ; CHEN RC ; SHI J ; LIU H ; HOU XJ ; WANG Q ; WANG H.** Improved production of holotoxin Stx2 with biological activities by using a single-promoter vector and an auto-induction expression system. *Protein Expr. Purif.,* 2009, vol. 67 (2), 169-174